# EUROPEAN PATENT SPECIFICATION

(11) **EP 4 362 678 B1**
(45) Date of publication and mention of the grant of the patent: **01.07.2026**
(21) Application number: 22761732.1
(22) Date of filing: 02.08.2022
(51) Int. Cl.: A01N 31/08, A01N 37/36, A01P 1/00, A61K 8/34, A61K 8/35, A61K 8/365, A61K 8/92, A61Q 17/00, A61Q 19/10

(54) **PERSONAL CARE COMPOSITIONS**
KÖRPERPFLEGEZUSAMMENSETZUNGEN
COMPOSITIONS DE SOINS PERSONNELS

(30) Priority: 03.08.2021 CN 202110884865
(43) Date of publication of application: 08.05.2024
(73) Proprietor: Colgate-Palmolive Company, New York, NY 10022 (US)
(72) Inventor: LIAO, Yuxi, Guangzhou, Guangdong 510730 (CN); LU, Xiaojing, Guangzhou, Guangdong 510730 (CN); SHI, Manying, Guangzhou, Guangdong 510730 (CN); ZHANG, Yuchen, Guangzhou, Guangdong 510730 (CN); WANG, Xiaojun, Guangzhou, Guangdong 510730 (CN)
(74) Representative: Wichmann, Hendrik
(86) International application number: PCT/US2022/039154
(87) International publication number: WO 2023/014706

(56) References cited:
- WO-A1-2013/103556
- WO-A1-2015/138479
- US-A1- 2014 242 198
- US-A1- 2019 327 963
- DATABASE GNPD [online] MINTEL; 14 November 2017 (2017-11-14), ANONYMOUS: "Daily Intimate Lotion", XP093002783, retrieved from https://www.gnpd.com/sinatra/recordpage/5237711/ Database accession no. 5237711
- PEI RUI-SONG ET AL: "Evaluation of Combined Antibacterial Effects of Eugenol, Cinnamaldehyde, Thymol, and Carvacrol against? E. coli ?with an Improved Method", JOURNAL OF FOOD SCIENCE, vol. 74, no. 7, 1 September 2009 (2009-09-01), US, pages M379 - M383, XP093003586, ISSN: 0022-1147, DOI: 10.1111/j.1750-3841.2009.01287.x

## Description

### CROSS-REFERENCE TO RELATED APPLICATIONS

The instant application is a U.S. Non-Provisional Application which claims priority to Chinese Application No. 202110884865.9, filed on August 3, 2021.

### TECHNICAL FIELD

The present application relates to a personal care composition, in at least one aspect an alcohol-free personal care composition, e.g., hand sanitizers, and to a method of reducing topical bacteria on a subject's skin.

### BACKGROUND

Personal hygiene garners a significant amount of attention, in particular hand hygiene. Effectively cleaning one's hands may require hand washing or hand sanitizer several times per day. Accordingly, hand sanitizer is a more convenient and effective way for hand cleaning in most occasions. Currently, the majority of hand sanitizers or sprays in the market are alcohol based. Unfortunately, many alcohol-based sanitizers may lead to dryness and can be especially harsh on the user's skin. A number of hand sanitizers currently on the market need to be used many times, throughout the day, in order to be effective. Consequently, with side-effects such as dryness, the need to use a hand sanitizer multiple times a day can be problematic for consumers eager to have a safer and milder product that can also deliver effective antibacterial functions. However, it is not necessarily straightforward to develop a natural, safe and effective alcohol-free hand sanitizer.

US2014242198 A1 relates to antimicrobial compositions such as hand disinfectants, and discloses various compositions comprising thymol and lactic acid The document shows alcohol-free hand disinfectant foams comprising thymol, lactic acid and citric acid (lemon juice) as well as caprylyl/capryl glucoside. The compositions are active against the bacteria *S.aureus* and *E.coli.*

Accordingly, there is a need in the market to develop a non-alcohol natural hand sanitizer, for example, a foam or spray, that can be safe and effective but that does not have some of the potential drawbacks or negative attributes that are common in many alcohol-based sanitizers.

### BRIEF SUMMARY

The disclosure provides for a personal care composition, e.g., a hand sanitizer, that comprises a combination of natural acids and plant essential oils as active antibacterial ingredients. The combinations described herein can unexpectedly provide strong, and relatively quick effects to neutralize or reduce various bacterial species without the use of an alcohol base. Moreover, the active materials used in the formulas of the disclosure have the benefit of being naturally derived.

The higher naturality and safety of the personal care compositions of the disclosure is advantageous in that it can meet a market need for personal care compositions (e.g., hand sanitizers) that are both efficacious, contain natural ingredients and do not contain alcohol. NB: The references to the methods of treatment by therapy or surgery or in vivo diagnosis methods in this description are to be interpreted as references to compounds, pharmaceutical compositions and medicaments of the present invention for use in those methods.

In one aspect, the invention provides a personal care composition (e.g., a hand sanitizer) which comprises an essential oil system and an organic acid, wherein the essential oil system comprises thymol, carvone, and wherein the organic acid comprises lactic acid. In still further embodiments, the composition is free of any alcohol. In some embodiments, the composition is selected from the group consisting of: hand sanitizer, liquid soap, liquid hand soap, shower gel, body wash, shampoo, or hair conditioner.

In another aspect, the invention provides a method of depositing a topically active compound on the skin, comprising applying an effective amount of any of personal care compositions as disclosed herein to the skin.

In some embodiments, the composition comprises an alkyl glucoside as a co-surfactant.

Further areas of applicability of the present disclosure will become apparent from the detailed description provided hereinafter. It should be understood that the detailed description and specific examples, while indicating some typical aspects of the disclosure, are intended for purposes of illustration only and are not intended to limit the scope of the disclosure.

### DETAILED DESCRIPTION

The following description of various typical aspect(s) is merely exemplary in nature and is in no way intended to limit the disclosure, its application, or uses.

As used throughout, ranges are used as shorthand for describing each and every value that is within the range. Any value within the range can be selected as the terminus of the range.

Unless otherwise specified, all percentages and amounts expressed herein and elsewhere in the specification should be understood to refer to percentages by weight relative to the total composition. The amounts given are based on the active weight of the material.

The present disclosure relates to alcohol-free personal care compositions that effectively deposit topically active natural antibacterial compounds (e.g., carvone and thymol) on the skin. For example, it has been found that the addition of thymol and carvone, essential oils, in combination with lactic acid, a carboxylic acid, into an alcohol-free product leads to unexpected benefits with respect to bacterial reduction, e.g., with respect to reduction of *S. aureus* and *E. coli* bacteria. Without being bound by theory, it is believed that the alcohol-free compositions of the disclosure (e.g., alcohol-free hand sanitizers) can incorporate natural antibacterial ingredients and still function at parity with comparable hand sanitizers that utilize an alcohol base.

The present invention provides, in an aspect, a personal care composition (Composition 1.0), (e.g., a hand sanitizer), comprising:
- An effective amount of an essential oil system, wherein the essential oil system comprises an essential oil comprising at least thymol and carvone, and optionally eugenol, carvacrol, tea tree oil, and combinations thereof; and
- A carboxylic acid source, and wherein the carboxylic acid source comprises lactic acid and/or citric acid.

For example, the disclosure includes:
1.1. The personal care composition of Composition 1.0, comprises: thymol, carvone, and eugenol.

A personal care composition of composition 1.0, wherein the essential oil system comprises: thymol, carvone, and tea tree oil.

A personal care composition of composition 1.0, wherein the essential oil system comprises: thymol, carvone, and carvacrol.

A personal care composition of composition 1.0, wherein the essential oil system comprises: thymol, carvone, eugenol, and tea tree oil.

A personal care composition of composition 1.0, wherein the essential oil system comprises: thymol, carvone, carvacrol, eugenol, and tea tree oil.

Any of the preceding personal care compositions, wherein the essential oil system further comprises an essential oil selected from the group consisting of: limonene, pinene, camphene, cymene, citronellol, geraniol, nerol, linalool, terpineol, rhodinol, borneol, isoborneol, menthone, camphor, safrole, isosafrole, menthol, geraniol, vertenone, eucalyptol, pinocarvone, cedrol, anethol, hinokitiol, berberine, ferulic acid, cinnamic acid, methyl salicylic acid, methyl salicylate, terpineol, rosemary extract, tea extract, magnolia extract, menthol, eucalyptol, citral, catechol, epigallocatechin gallate, epigallocatechin, gallic acid, miswak extract, sea-buckthorn extract, isoeugenol, and combinations thereof.

Any of the preceding personal care compositions wherein the thymol is present in an amount from 0.05% - 2% by wt. of the total composition (e.g., from 0.075% - 1% by wt.) (e.g., about 0.1% by wt.).

Any of the preceding personal care compositions wherein the carvone is present in an amount from 0.01% - 2% by wt. of the total composition (e.g., about 0.05% by wt.).

Any of the preceding personal care compositions wherein the eugenol is present in an amount from 0.1% - 2% by wt. of the total composition (e.g., about 0.1% by wt.).

Any of the preceding personal care compositions wherein the carvacrol is present in an amount from 0.01% - 2% by wt. of the total composition (e.g., about 0.05% by wt.).

Any of the preceding personal care compositions wherein the tea tree oil is present in an amount from 0.1% - 2% by wt. of the total composition (e.g., about 0.3% by wt.).

Any of the preceding personal care compositions wherein the essential oil system is present in an amount from 0.075 - 4% by wt. of the total composition (e.g., from 0.075% - 0.2%) (e.g., about 0.15%).

Any of the preceding personal care compositions, wherein the composition comprises one or more surfactant(s).

Any of the preceding personal care compositions, wherein the surfactant comprises an alkyl glucoside.

Any of the preceding personal care compositions, wherein the alkyl glucoside is present in an amount of from 0.5% to 9%, e.g., from 0.75% to 8%, from 0.75% to 7%, from 0.75% to 6%, by weight of the composition, optionally wherein the alkyl glucoside is present in an amount of about 1% by weight of the composition.

Any of the preceding personal care compositions, wherein the alkyl glucoside is C₈₋₂₅ alkyl glucoside, e.g., C₈₋₁₈ alkyl glucoside, C₁₀₋₁₈ alkyl glucoside or C₁₀₋₁₆ alkyl glucoside, optionally wherein the alkyl glucoside is selected from decyl glucoside, caprylyl/capryl glucoside, lauryl glucoside, coco-glucoside, octyl glucoside, cetearyl glucoside, cetyl glucoside, hexadecyl glucoside, arachidyl glucoside, and a combination thereof.

Any of the preceding compositions, wherein the alkyl glucoside is decyl glucoside (e.g., at about 1% by wt.)

Any of the preceding personal care compositions, wherein the surfactant comprises an effective amount of a zwitterionic surfactant (e.g., a betaine zwitterionic surfactant) (e.g., cocamidopropyl betaine).

Any preceding personal care composition, wherein the zwitterionic surfactant is a betaine zwitterionic surfactant (e.g., from 0.1% - 5% by wt. of the total composition) (e.g., 0.5% - 4% by wt. of the total composition) (e.g., about 3% by wt. of the total composition). The preceding personal care composition, wherein the betaine zwitterionic surfactant is a C8-C16 aminopropyl betaine (e.g., cocamidopropyl betaine)

The preceding personal care composition wherein the C8-C16 aminopropyl betaine is cocamidopropyl betaine.

The preceding personal care composition wherein the cocamidopropyl betaine, is present in an amount of from 0.5% to 4% by wt of the total composition.

The preceding personal care composition, wherein the cocamidopropyl betaine is from 1% to 4% by wt of the total composition.

The preceding personal care composition wherein the cocamidopropyl betaine is from 1.5% to 3.5% (e.g., about 3% by wt. of the total composition).

Any of the preceding personal care compositions wherein the composition comprises cocamidopropyl betaine and decyl glucoside in a wt% ratio of (e.g., wt%) is from 3:1 to 0.5:1 (e.g., 1:1)

Any of the preceding personal care compositions, wherein the total amount of surfactants present in the composition is from 0.5% to 5%, e.g., about 1% by weight of the composition.

Any of the preceding personal care composition wherein the carboxylic acid source comprises lactic acid (e.g., from 0.05% - 2% by wt. of the composition).

Any of the preceding personal care compositions wherein the carboxylic acid source comprises citric acid (e.g., from 0.05% - 2% by wt. of the composition).

Any of the preceding personal care compositions wherein the carboxylic acid source further comprises glycolic acid, or acetic acid, or succinic acid, or fumaric acid, or combinations thereof.

Any of the preceding personal care compositions, wherein the composition comprises a thickener.

Any of the preceding personal care compositions, wherein the thickener comprises a gum, optionally selected from xanthan gum, carrageenan and a combination thereof.

Any of the preceding personal care compositions, wherein the composition comprises a humectant, optionally wherein the humectant is selected from glycerin, sorbitol, xylitol, 1, 3-propanediol, propylene glycol, polyethylene glycol and a combination thereof.

The preceding personal care composition, wherein the amount of surfactant in the composition is from 1% - 15% by wt. of the total composition.

Any of the preceding personal care compositions, wherein the composition comprises water (e.g., greater than 70% water by wt. of the total composition) (e.g., from 65% - 95% by wt. of the total composition) (e.g., from 65% - 85% by wt. of the total composition) (e.g., from 65% - 75% by wt. of the total composition).

Any of the preceding personal care compositions comprising a fragrance and/or color.

Any of the preceding personal care compositions comprising an antibacterial agent selected from herbal extracts, bisguanide antiseptics (e.g., chlorhexidine, alexidine or octenidine), quaternary ammonium compounds (e.g., cetylpyridinium chloride (CPC), benzalkonium chloride, tetradecylpyridinium chloride (TPC), N-tetradecyl-4-ethylpyridinium chloride (TDEPC)), phenolic antiseptics, hexetidine, octenidine, sanguinarine, povidone iodine, delmopinol, salifluor, metal ions (e.g., zinc salts, for example, zinc citrate, stannous salts, copper salts, iron salts), sanguinarine, propolis, phthalic acid and its salts, monoperthalic acid and its salts and esters, ascorbyl stearate, oleoyl sarcosine, alkyl sulfate, dioctyl sulfosuccinate, salicylanilide, domiphen bromide, delmopinol, octapinol and other piperidino derivatives, nicin preparations, chlorite salts; and mixtures of any of the foregoing.

Any of the preceding personal care compositions comprising an antiinflammatory compound.

Any of the preceding personal care compositions, wherein the composition is free or substantially free of alcohol.

Any of the preceding personal care compositions, wherein the composition (e.g., a hand sanitizer) comprises:
- Thymol (e.g., from 0.05% - 2% by wt.) (e.g., 0.1% by wt.)
- Carvone (e.g., from 0.01% - 2% by wt.) (e.g., 0.05% by wt.)
- Decyl glucoside (e.g., from 0.05% - 2% by wt.)
- 1, 3-propanediol (e.g., from 0.5% - 4% by wt.)
- Sorbitol (e.g., from 0.5% - 6% by wt.)
- Glycerin (e.g., 0.05 - 2%)
- Xylitol (e.g., from 0.5% - 4% by wt.) (e.g., about 2% by wt.);
- Lactic acid (e.g., from 0.05 - 2% by wt.)
- Water

Any of the preceding compositions, wherein the composition further comprises a gelling agent, or an antioxidant, or a fragrance, or a combination thereof.

Any of the preceding compositions, wherein the composition is selected from: a hand sanitizer, a liquid soap, liquid hand soap, shower gel, body wash, shampoo, and hair conditioner.

Any of the preceding composition wherein the personal care composition is a hand sanitizer.

Any of the preceding compositions wherein the pH of the composition is from 3 - 5 (e.g., from 3.25 - 4.25) (e.g., from 3.5 - 4) (e.g. about 3.6) (e.g., about 3.95)

Any of the preceding compositions wherein the composition is in an amount of effective to reduce *S.Aureus and*/*or E.coli.*

Any of the preceding compositions, wherein the essential oil system comprises a ratio (by wt.%) of thymol (by wt.%) to carvone (by wt.%) of 5:1 to 1:5 (e.g., 2:1).

Any of the preceding compositions, wherein the essential oil system comprises a ratio (by wt.%) of thymol (by wt.%) to eugenol (by wt.%) of 5:1 to 1:5 (e.g., 1:1).

Any of the preceding compositions, wherein the essential oil system comprises a ratio (by wt.%) of thymol (by wt.%) to carvacrol (by wt.%) of 5:1 to 1:5 (e.g., 2:1).

Any of the preceding compositions, wherein the composition comprises thymol (e.g., about 0.1% by wt.) and carvone (e.g., about 0.5% by wt.), and either citric acid or lactic acid, and wherein the composition reduces a *E. coli* population by at least 95% (e.g., about 99% reduction) after about 30 seconds or 60 seconds of contact.

Any of the preceding compositions comprising thymol (e.g., from 0.05% - 2% by wt.) (e.g., 0.1% by wt.), carvone (e.g., from 0.01% - 2% by wt.) (e.g., 0.05% by wt.), and lactic acid (e.g., from 0.05 - 2% by wt.).

Any of the preceding compositions, wherein the composition comprises xylitol in an amount from 0.5% - 5% by wt.

The present disclosure provides, in another aspect, a personal care composition e.g., any of Composition 1.0 et seq, for use in reducing bacteria (e.g., *E*. *coli* and/or *S. aureus)* on the skin of a subject.

In some aspects, the present disclosure provides a method of reducing topical bacteria on a subject's skin, wherein the method comprises administering any of Composition 1.0 et seq to the subject's skin.

In some aspects, the present disclosure provides a method of reducing topical bacteria (e.g., *E. coli* and/or *S. aureus)* on a subject's skin, wherein the method comprises administering any of Composition 1.0 et seq to the subject's skin and wherein any of Composition 1.0 et seq is applied to the skin in order to reduce the amount of *E. coli* and/or *S. aureus* bacteria. In some aspects the personal care composition (e.g., any of Composition 1.0 et seq) is applied for at least 30 seconds in order to obtain a reduction of at least 90% of the amount of *E. coli.* In still a further aspect, the personal care composition (e.g., any of Composition 1.0 et seq) is applied for at least 60 seconds in order to obtain a reduction of at least 95% of the amount of *E. coli.*

In some embodiments, any of Composition 1.0 et seq, comprises an oil selected from sunflower seed oil, olive oil, shea butter, jojoba oil, almond oil, grape seed oil, rose hip seed oil, mink oil, castor oil, soybean oil, mineral oil, and a combination thereof.

The composition of the present disclosure, e.g., any of Composition 1.0 et seq., may be any type of personal care composition. In certain embodiments, the composition is any composition that can be formulated into topical skin care formulations suitable for application to skin (e.g., a hand sanitizer). Examples of such personal care compositions include, but are not limited to: skin care compositions (e.g., hand sanitizer), antiperspirants, deodorants, body washes, creams, shower gels, bar soaps, shampoo, hair conditioners, and cosmetics. In some embodiments, the composition is a rinse off product (liquid soap, liquid hand soap, shower gel, body wash, shampoo, or hair conditioner, etc.). The composition can comprise a single phase or can be a multi-phase system, for example a system comprising a polar phase and an oil phase, optionally in the form of a stable emulsion. The composition can be liquid, semi-solid or solid. The formulation can be provided in any suitable container such as an aerosol can, tube or container with a porous cap, roll-on container, bottle, container with an open end, etc.

Water may be present in the composition. Water employed in the preparation of commercial personal care compositions should be deionized and free of organic impurities. Water commonly makes up the balance of the compositions and includes about 10% to about 95%, or about 10% to about 80%, by weight of the personal care compositions. This amount of water includes the free water which is added plus that amount which is introduced with other materials such as glycerin, sorbitol or any components of the invention.

In some embodiments the disclosure, e.g., any of Composition 1.0 et seq, further provides a alcohol-free personal care formulation comprising humectants, antioxidants, pH adjusters, vitamins, fragrances, thickeners, oils, and water.

In some embodiments of the disclosure, lactic acid, sodium hydroxide, and citric acid can be used to adjust the pH of the composition.

In some embodiments, any of Composition 1.0 et seq comprises one or more surfactant(s). Suitable surfactants may be zwitterionic or nonionic. In some embodiments, the surfactant may comprise an alkyl glucoside. Alkyl glucoside is a compound produced by combining a sugar such as glucose with a fatty alcohol. Alkyl refers to an unbranched or branched carbon chain. In some embodiments, the alkyl group is unbranched. The alkyl group may be saturated or unsaturated. In some embodiments, the alkyl group is saturated. For example, any of Composition 1.0 et seq can comprise decyl glucoside. The structure of decyl glucoside is shown below:

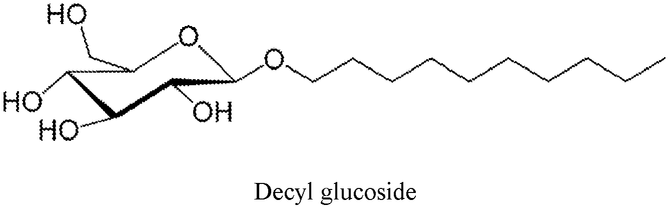

Alkyl glucoside may be C₈₋₂₅ alkyl glucoside, e.g., C₈₋₁₈ alkyl glucoside, C₁₀₋₁₈ alkyl glucoside or C₁₀₋₁₆ alkyl glucoside. In some embodiments, the alkyl glucoside is selected from decyl glucoside, caprylyl/capryl glucoside, lauryl glucoside, coco-glucoside, octyl glucoside, cetearyl glucoside, cetyl glucoside, hexadecyl glucoside, arachidyl glucoside, and a combination thereof. In some embodiments, the alkyl glucoside is selected from decyl glucoside, caprylyl/capryl glucoside and a combination thereof. In some embodiments, the weight ratio of caprylyl/capryl glucoside to decyl glucoside present in the composition is 2 to 1 or 4 to 1, e.g., about 3 to 1. In some embodiments, the alkyl glucoside is present in an amount of from 0.5% to 10% by weight of the composition.

In some embodiments, any of Composition 1.0 et seq may comprise a betaine zwitterionic surfactant, optionally together with an alkyl glucoside. The betaine zwitterionic surfactant may be a C₈-C₁₆ aminopropyl betaine, e.g., cocamidopropyl betaine. In some embodiments, the co-surfactant may comprise a non-ionic block copolymer, optionally together with an alkyl glucoside. The non-ionic block copolymer may be a poly(propylene oxide)/poly(ethylene oxide) copolymer. In some embodiments, the copolymer has a polyoxypropylene molecular mass of from 3000 to 5000 g/mol and a polyoxyethylene content of from 60 to 80 mol%. In some embodiments, the non-ionic block copolymer is a poloxamer. In some embodiments, the non-ionic block copolymer is selected from: Poloxamer 338, Poloxamer 407, Poloxamer, 237, Poloxamer, 217, Poloxamer 124, Poloxamer 184, Poloxamer 185, and a combination of two or more thereof. In some embodiments, the copolymer is Poloxamer 407. In some embodiments, the co-surfactant may comprise a betaine zwitterionic surfactant and a non-ionic block copolymer, optionally together with an alkyl glucoside.

In some embodiments, the personal care composition, e.g., any of Composition 1.0 et seq, may be free or substantially free of sodium lauryl sulfate (SLS) and/or sodium lauryl ether sulfate (SLES). In some embodiments, the composition may be free or substantially free of alkyl sulfate salts. In some embodiments, the alkyl sulfate salts are C₁₋₂₅ alkyl sulfate which may be saturated or unsaturated, and unbranched or branched. In some embodiments, the personal care composition may be free or substantially free of alkyl aryl sulfonate salts, e.g., alkyl benzene sulfonate salts, e.g., sodium dodecyl benzene sulfonate. In some embodiments, the personal care composition may be free or substantially free of alkyl sulfate salts and alkyl aryl sulfonate salts. In some embodiments, the composition may be free or substantially free of sulfate.

As used herein, "personal care composition" refers to a product that in the ordinary course of usage can be applied to the skin or contacts a body surface to provide a beneficial effect. Body surface includes skin, for example dermal or mucosal. Body surface can also include structures that are associated with the body surface, e.g., hair, teeth, or nails. Examples of personal care compositions may include a product applied to a human body for improving appearance, cleansing, odor control, and/or general aesthetics.

As used herein, "substantially free" of a material may refer to a composition where the material is present in an amount of less than 0.1 weight %, less than 0.05 weight %, less than 0.01 weight %, less than 0.005 weight %, less than 0.001 weight %, or less than 0.0001 weight % based on a total weight of the composition.

As used herein, "carvone" refers to a compound that is a member the terpenoid family of chemical compounds and has the following structure:

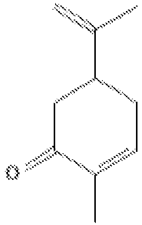

As used herein, "carvone" can refer to either *R*-(-)-carvone or S-(+)-carvone.

As used herein, "thymol" can refer to 2-isopropyl-5-methylphenol and is a natural monoterpenoid phenol derivative of p-Cymene, C₁₀H₁₄O, isomeric with carvacrol, found in oil of thyme, and extracted from *Thymus vulgaris* (common thyme). "Thymol" has the following structure:

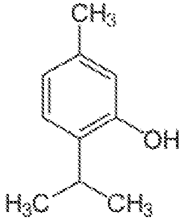

In some embodiments, the total amount of surfactants present in the personal care composition may be from 0.5% to 5%, e.g., from 1% to 4%, e.g., about 2% by weight of the composition.

The surfactant system disclosed in this disclosure increases the deposition of topically active compounds on the skin when the surfactant system is used in personal care compositions, e.g., skin care compositions. Topically active compounds encompass a wide range of materials, including antibacterial agents, vitamins, medicaments, fragrance materials, antioxidants, antiperspirant actives, deodorant actives, and other skin-care ingredients. In some embodiments, the personal care composition of the present disclosure comprises a vitamin. Illustrative vitamins may be or include, but are not limited to, vitamin C, vitamin D, vitamin E, vitamin K, and a combination thereof. In some embodiments, the composition comprises vitamin E, e.g., vitamin E acetate. In some embodiments, the vitamin may be present in an amount of from 0.05% to 1%, e.g., from 0.05% to 0.5%, from 0.05% to 0.3%, from 0.05% to 0.2%, from 0.05% to 0.15%, or about 0.1%, by weight of the composition.

In some embodiments, the personal care composition of the invention can comprise: glycols including propylene glycol, dipropylene glycol, tripropylene glycol and mixtures thereof; glycerides including mono-, di- and triglycerides; medium to long chain organic acids, alcohols and esters; surfactants including emulsifying and dispersing agents; amino acids including glycine; structurants including thickeners and gelling agents, for example polymers, silicates and silicon dioxide; emollients; fragrances; and colorants including dyes and pigments.

The personal care composition of the disclosure, e.g., any of Composition 1.0 et seq, may contain emollients in any desired amount to achieve a desired emollient effect. Emollients are known in the art and are used to impart a soothing effect on the skin. Non-volatile emollients are preferable. Classes of non-volatile emollients include non-silicone and silicone emollients. Non-volatile, non-silicone emollients include C₁₂₋₁₅ alkyl benzoate. The non-volatile silicone material can be a polyethersiloxane, polyalkyarylsiloxane or polyethersiloxane copolymer. An illustrative non-volatile silicone material is phenyl trimethicone. Examples include, but are not limited to, PPG-14 butyl ether, PPG-3 myristyl ether, secondary alcohol ethoxylates, stearyl alcohol, stearic acid and salts thereof, glyceryl monoricinoleate, isobutyl palmitate, glyceryl monostearate, isocetyl stearate, sulphated tallow, oleyl alcohol, propylene glycol, isopropyl laurate, mink oil, sorbitan stearate, cetyl alcohol, hydrogenated castor oil, stearyl stearate, hydrogenated soy glycerides, isopropyl isostearate, hexyl laurate, dimethyl brassylate, decyl oleate, diisopropyl adipate, n-dibutyl sebacate, diisopropyl sebacate, 2-ethyl hexyl palmitate, isononyl isononanoate, isodecyl isononanoate, isotridecyl isononanoate, 2-ethyl hexyl palmitate, 2-ethyl hexyl stearate, Di-(2-ethyl hexyl)adipate), Di-(2-ethyl hexyl)succinate, isopropyl myristate, isopropyl palmitate, isopropyl stearate, octacosanol, butyl stearate, glyceryl monostearate, polyethylene glycols, oleic acid, triethylene glycol, lanolin, castor oil, sunflower seed oil, acetylated lanolin alcohols, acetylated lanolin, petrolatum, isopropyl ester of lanolin, fatty acids, mineral oils, butyl myristate, isostearic acid, palmitic acid, PEG-23 oleyl ether, olelyl oleate, isopropyl linoleate, cetyl lactate, lauryl lactate, myristyl lactate, quaternised hydroxy alkyl, aminogluconate, vegetable oils, isodecyl oleate, isostearyl neopentanoate, myristyl myristate, oleyl ethoxy myristate, diglycol stearate, ethylene glycol monostearate, myristyl stearate, isopropyl lanolate, paraffin waxes, glycyrrhizic acid, hydroxyethyl stearate amide. In some embodiments, the composition comprises an oil selected from sunflower seed oil, olive oil, shea butter, jojoba oil, almond oil, grape seed oil, rose hip seed oil, mink oil, castor oil, soybean oil, mineral oil, and a combination thereof. In certain embodiment, the composition comprises sunflower seed oil.

The personal care composition of the disclosure, e.g., any of Composition 1.0 et seq, may include one or more humectants. Humectants can reduce evaporation and also contribute towards preservation by lowering water activity and can also impart desirable sweetness or flavor to compositions. Illustrative humectants may be or include, but are not limited to, glycerin, propylene glycol, 1,3-propanediol, polyethylene glycol, sorbitol, xylitol, or the like, or any mixture or combination thereof. In some embodiments, the humectant is selected from glycerin, xylitol, sorbitol, 1,3-propanediol and a combination thereof.

In some embodiments the personal composition, e.g., any of Composition 1.0 et seq, may include thickeners. Illustrative thickeners may be or include, but are not limited to, colloidal silica, fumed silica, a cross-linked polyvinylpyrrolidone (PVP) polymer, cross-linked polyvinylpyrrolidone (PVP), or the like, or mixtures or combinations thereof. In some embodiments, the thickening system includes a cross-linked polyvinylpyrrolidone (PVP) polymer. Illustrative thickeners may also be or include, but are not limited to, carbomers (*e.g*., carboxyvinyl polymers), carrageenans (*e.g.,* Irish moss, carrageenan, iota-carrageenan, *etc.*)*,* high molecular weight polyethylene glycols, cellulosic polymers, hydroxyethylcellulose, carboxymethylcellulose, and salts thereof (*e.g.,* CMC sodium), natural gums (*e.g.,* karaya, xanthan, gum arabic, and tragacanth), colloidal magnesium aluminum silicate, or the like, or mixtures or combinations thereof. In some embodiments, the thickener comprises or is a gum, optionally selected from xanthan gum, carrageenan, and a combination thereof.

In some embodiments, the personal care composition of the disclosure, e.g., any of Composition 1.0 et seq, comprises one or more gelling agents. Examples of gelling agents include, but are not limited to, waxes, esters of fatty acid and fatty alcohol, triglycerides, partially or fully hydrogenated soybean oil, partially or fully hydrogenated castor oil, other partial or fully hydrogenated plant oils, stearyl alcohol, or other cosmetically acceptable materials, which are solid or semi-solid at room temperature and provide a consistency suitable for application to the skin.

Antioxidants may be added to the composition, preferably to act as ingredient protectants and for maintenance of long-term stability of the composition. Examples of antioxidants include, but are not limited to citric acid, butylated hydroxytoluene, pentaerythrityl tetra-di-t-butyl hydroxyhydrocinnamate.

In some embodiments, the personal care composition of the disclosure, e.g., any of Composition 1.0 et seq, comprises polymeric materials for thickening, such as polyamides, cellulose derivatives (e.g., hydroxypropylcellulose, hydroxypropyl methyl cellulose, etc.) and natural or synthetic gums, such as polyglycerides including agar, agarose, pectin, or guars or mixtures or combinations thereof. One class of materials worthy of attention for thickening a water-immiscible phase comprises derivatives of hydrolysed starch or other polysaccharides, including in particular esterified dextrins, such as dextrin palmitate. A further class of polymers that is particularly directed to structuring an oil phase containing a silicone oil comprises polysiloxane elastomers. Suspending agents such as silicas or clays such as bentonite, montmorillonite or hectorite, including those available under the trademark Bentone can also be employed to thicken liquid compositions according to the invention. The composition can be thickened with non-polymeric organic gellants, including selected dibenzylidene alditols (e.g., dibenzylidene sorbitol).

In some embodiments, the personal care composition of the disclosure, e.g., any of Composition 1.0 et seq, comprises a fragrance. Any fragrance suitable for personal care use may be incorporated into the personal care composition of the invention. Fragrances tend to be relatively volatile aroma compounds which are capable of entering the gas phase at skin surface temperature.

The personal care compositions of the disclosure may be manufactured using methods known in the art. In one aspect, the ingredients are combined and optionally heated where components need to be melted. The components are mixed. In one aspect there is a pre-mix process wherein the essential oil is mixed with at least one surfactant and humectant to ensure the components are completely in solution of the oil phase. Desirably, volatile materials such as fragrant materials are incorporated in the composition in the latter stages of a mixing cycle in order to avoid volatilization thereof. After mixing, the composition may be poured directly into the dispensers and the container capped to preserve the product until use.

In another aspect, the invention provides a method of depositing a topically active compound on the skin, comprising applying an effective amount of any of personal care compositions disclosed herein, e.g., any of Compositions 1 *et seq.,* to the skin.

### EXAMPLES

NB: Inventive examples are those according to the claims.

### Example 1

### Determination of Antibacterial Efficacy

Four basic personal care formulas are prepared according to **Table 1** below. Antibacterial efficacy of the formulas is generally detailed in Table 2.

**Table 1. Formulations 1- 4**

| **Ingredient [% by wt.]** | **Formulation** | | | |
|---|---|---|---|---|
| | **1** | **2** | **3** | **4** |
| Thymol | 0.1 | 0.1 | 0.1 | 0 |
| Eugenol | 0.1 | - | - | - |
| Carvone | - | - | 0.5 | - |
| Carvacrol | - | 0.05 | - | - |
| Tea Tree Oil | - | - | - | 0.3 |
| Lactic acid | 0.1 | 0.1 | 0.1 | 0.1 |
| Decyl glucoside | 1 | 1 | 1 | - |
| Cocamidopropyl Betaine (e.g., 30% solution) | - | - | - | 1 |
| Glycerin | - | 0.12 | 0.12 | 0.12 |
| 1,3-propanediol | 2 | 2 | 2 | 2 |
| xylitol | - | 2 | 2 | 2 |
| sorbitol (e.g., Sorbitol 50% solution) | 2 | 5 | 5 | 5 |
| Water | Q.S. (To balance) | Q.S. (To balance) | Q.S. (To balance) | Q.S. (To balance) |
| **Total** | 100% | 100% | 100% | 100% |
| **pH** | 3.64 | 3.59 | 3.61 | 3.14 |

**Table 2 - Antibacterial and Antiviral Assay**

| Prototype | | Contact Time | Bacterial | Killing Rate |
|---|---|---|---|---|
| #1 | 0.1% Thymol + 0.1% Eugenol | 30s | *S.aureus & E. coli* | 99.99% |
| | 0.1% Lactic acid | | | |
| | pH 3.64 | | | |
| #2 | 0.1% Thymol + 0.05% Carvacrol | | *S.aureus & E. coli* | 99.99% |
| | 0.1% Lactic acid | | | |
| | pH 3.56 | | | |
| #3 | 0.1% Thymol + 0.05% Carvone | | *S.aureus & E. coli* | 99.99% |
| | 0.1% Lactic acid | | | |
| | pH 3.61 | | | |
| #4 | 0.3% Tea Tree Oil | 90s | *S.aureus* | 99.99% |
| | 0.1% Lactic Acid | | *E. coli* | 99.99% |
| | pH 3.14 | | | |

As demonstrated in Table 2, Formulas 1- 4 demonstrate effective bacterial reduction for both *S.aureus & E.coli* after either 30 or 90 seconds of contact time. In further assays, Formula 1 further demonstrates 99.99% reduction in H1N1 virus after 30 seconds of contact time (data not shown in Table 2).

### Example 2

### Combination of a carboxylic acid and essential oils delivers an improvement in antibacterial reduction

**Table 3 - Formulas varying the combination of thymol and carvone with either lactic acid or citric acid:**

| | | **Citric Acid (Formula A)** | **Lactic Acid (Formula B)** | **Essential Oil (Formula C)** | **Citric Acid & Essential Oil (Formula D)** | **Lactic Acid & Essential Oil (Formula E)** |
|---|---|---|---|---|---|---|
| Thymol | | - | - | 0.1 | 0.1 | 0.1 |
| Carvone | | - | - | 0.05 | 0.05 | 0.05 |
| Lactic acid | | - | 0.1 | - | - | 0.1 |
| Citric Acid | | 0.1 | - | - | 0.1 | - |
| Glycerin | | - | - | 0.12 | 0.12 | 0.12 |
| APG | | 1 | 1 | 1 | 1 | 1 |
| 1,3-propanediol | | 2 | 2 | 2 | 2 | 2 |
| Xylitol | | 2 | 2 | 2 | 2 | 2 |
| Sorbitol | | 5 | 5 | 5 | 5 | 5 |
| Water | | q.s. (To balance) | q.s. (To balance) | q.s. (To balance) | q.s. (To balance) | q.s. (To balance) |

| **Antibacterial Effect** | | | | | | |
|---|---|---|---|---|---|---|
| *S.aureus* | 30s | 57.688% | 58.052% | 99.931% | >99.999% | >99.999% |
| | 60s | 78.607% | 76.061% | 99.956% | >99.999% | >99.999% |
| *E. Coli* | 30s | -23.872% | 5.704% | 61.039% | >99.999% | >99.999% |
| | 60s | 29.706% | -5.954% | 95.027% | >99.999% | >99.999% |

Formula samples, e.g., A - E listed in Table 3, demonstrate an improved benefit with the combination of both an essential oil, e.g., thymol and carvone, in combination with citric acid or lactic acid. For example, formula samples D and E with thymol and carvone, and either citric acid or lactic acid, unexpectedly improve upon the bacterial reduction of *E. coli* at both the 30s and 60s time points (>99.999% reduction in both) when compared to formula sample C that only contains thymol and carvone (and not lactic acid or citric acid) at both the 30s (61.039% reduction) and 60s (95.027%) time points. Similarly, formula samples A and B - which contain only citric acid or lactic acid and do not contain a combination of both an essential oil, e.g., thymol and carvone - had a lower reduction of *E. coli* at both the 30s and 60s time points when compared to formula samples C, D and E.

### Example 3

***The amount of thymol impacts antibacterial efficacy in E. coli***

**Table 4 - Formulations varying the amount of thymol (amounts % by wt.)**

| | | Formula D (by wt%) | Formula E (by wt%) | Formula F (by wt%) |
|---|---|---|---|---|
| | Thymol | 0.05 | 0.05 | 0.1 |
| | Carvone | - | 0.05 | 0.05 |
| | Lactic acid | 0.1 | 0.1 | 0.1 |
| | Glycerin | 0.12 | 0.12 | 0.12 |
| | CAP Betaine | 1 | 1 | 1 |
| | 1,3-propanediol | 2 | 2 | 2 |
| | Xylitol | 2 | 2 | 2 |
| | Sorbitol | 5 | 5 | 5 |

| **Antibacterial Effect** | | | | |
|---|---|---|---|---|
| *S. aureus* | 30s | 99.39% | 99.98% | 99.997% |
| *S. aureus* | 60s | 99.91% | 99.98% | >99.999% |
| *E. coli* | 30s | 71.56% | 62.72% | >99.999% |
| *E. coli* | 60s | 84.76% | 92.67% | >99.999% |

Formula samples, e.g., D - F listed in Table 4, demonstrate an improved benefit with the combination of both thymol and carvone, in combination with lactic acid, where the amount of thymol is 0.1% by wt. For example, samples with thymol (0.1%) and carvone (0.05%), and lactic acid, unexpectedly improve upon the bacterial reduction of *E. coli* at both the 30s and 60s time points (>99.999% reduction in both) when compared to samples that contain thymol (0.05%) and carvone (0.5%) at both the 30s (61.039% reduction) and 60s (95.027%) time points. Note, when comparing Formula D and Formula E, that the reduction of *E.coli* at the 30s time point is *decreased* despite the fact that the amount of thymol (0.05%) is consistent and carvone (0.05%) is added to Formula E. The reduction in *E. coli* at the 30s time point then *increases* in Formula F with the increase of thymol (0.1%) but keeping all other ingredient amounts the same.

### Example 4

**Table 5 - pH of the formulation affects antibacterial efficacy in E. coli (*Ingredients listed by (wt% relative to the total composition)**

| | | | | | | |
|---|---|---|---|---|---|---|
| pH | | 3.61 | 3.97 | 6.08 | 8.71 | 10.10 |

| Ingredients (by wt.%) | | Formula G | Formula H | Formula I | Formula J | Formula K |
|---|---|---|---|---|---|---|
| Thymol | | 0.1 | 0.1 | 0.1 | 0.1 | 0.1 |
| Carvone | | 0.05 | 0.05 | 0.05 | 0.05 | 0.05 |
| Lactic acid | | 0.1 | 0.05 | 0.025 | 0.02 | - |
| Glycerin | | 0.12 | 0.12 | 0.12 | 0.12 | 0.12 |
| Alkyl polyglucoside (APG) | | 1 | 1 | 1 | 1 | 1 |
| 1,3-Propanediol | | 2 | 2 | 2 | 2 | 2 |
| Xylitol | | 2 | 2 | 2 | 2 | 2 |
| Sorbitol | | 5 | 5 | 5 | 5 | 5 |

| **Antibacterial Effect** | | | | | | |
|---|---|---|---|---|---|---|
| *S.aureus* | 30s | >99.999% | >99.999% | 98.773% | 99.486% | 99.931% |
| | 60s | >99.999% | >99.999% | 99.955% | 99.974% | 99.956% |
| *E.Coli* | 30s | >99.999% | >99.999% | 83.766% | 54.708% | 61.039% |
| | 60s | >99.999% | >99.999% | 95.557% | 81.786% | 95.027% |

Controlling for the amounts of thymol and carvone, as well as the APG, xylitol, sorbitol and 1,3-Propanediol, Table 5 indicates that once pH is greater than 6 there are reductions in the antibacterial efficacy of the more basic formulations.

### Example 5

**Table 6 - Inclusion of xylitol can increase the antibacterial efficacy in reducing E.coli**

| Ingredients (wt%) | | **Formula L** | **Formula M** |
|---|---|---|---|
| Thymol | | 0.05 | 0.05 |
| Eugenol | | 0.05 | 0.05 |
| Lactic acid | | 0.1 | 0.1 |
| Glycerin | | 0.12 | 0.12 |
| Cocamidopropyl Betaine | | 1 | 1 |
| 1,3- Propanediol | | 2 | 2 |
| Xylitol | | - | 2 |
| Sorbitol | | 5 | 5 |

| **Antibacterial Effect** | | | |
|---|---|---|---|
| *S.aureus* | 30s | 99.98% | 99.98% |
| | 60s | 99.98% | 99.923% |
| *E.Coli* | 30s | 64.910% | 90.600% |
| | 60s | 90.560% | 99.999% |

| | | | |
|---|---|---|---|
| ***(*Ingredients listed by wt% relative to the total composition)*** | | | |

Controlling for the amounts of thymol and eugenol, as well as the APG, xylitol, sorbitol and 1,3-Propanediol, Table 6 indicates that formulas with xylitol demonstrate a greater antibacterial effect with respect to *E. coli* than similar formulations that do not contain xylitol.

### Example 6

### Representative formula

In one aspect, Table 7 is a representative formula of a hand sanitizer of the present disclosure:

**Table 7**

| Ingredients | Amount (wt%) |
|---|---|
| Thymol | 0.05% - 0.5% |
| Carvone | 0.01% - 0.5% |
| Lactic acid | 0.05% - 0.5% |
| Glycerin | 0.05% - 0.5% |
| Alkyl polyglucoside | 0.5% - 2% |
| 1,3- Propanediol | 1% - 5% |
| Xylitol | 1% - 5% |
| Sorbitol (Sorbitol 50% solution) | 1 - 10% |
| Water | q. s. (To balance) |
| Total | 100% |

The present disclosure has been described with reference to exemplary embodiments. Although a limited number of embodiments have been shown and described, it will be appreciated by those skilled in the art that changes may be made in these embodiments without departing from the principles of the preceding detailed description. It is intended that the present disclosure be construed as including all such modifications and alterations insofar as they come within the scope of the appended claims.

## Claims

1. A personal care composition comprising:
a. an effective amount of an essential oil system, wherein the essential oil system comprises thymol, carvone and an optional essential oil selected from the group consisting of eugenol, carvacrol, tea tree oil, and combinations thereof; and
b. a carboxylic acid source, and wherein the carboxylic acid source comprises lactic acid and/or citric acid.

2. The personal care composition of claim 1, wherein the essential oil system comprises: thymol, carvone, and eugenol; thymol, carvone, and tea tree oil; thymol, carvone, and carvacrol; thymol, carvone, eugenol and tea tree oil; or thymol, carvone, carvacrol, eugenol and tea tree oil.

3. The personal care composition of any of the preceding claims, wherein the essential oil system comprises tea tree oil and the carboxylic acid source comprises lactic acid.

4. The personal care composition of any of the preceding claims, wherein the thymol is present in an amount from 0.05% - 2% by wt. of the total composition.

5. The personal care composition of any of the preceding claims, wherein the essential oil system is present in an amount from 0.075 - 4% by wt. of the total composition, in particular from 0.075% - 0.2%, more particularly about 0.15%.

6. The personal care composition of any of the preceding claims comprising a surfactant, wherein the surfactant comprises an alkyl glucoside, and/or wherein the carboxylic acid source comprises lactic acid.

7. The personal care composition of claim 6, wherein the alkyl glucoside, if present, is decyl glucoside.

8. The personal care composition of claim 7, wherein the lactic acid, if present, is present from 0.05% - 2% by wt. of the composition.

9. The personal care composition of any of the preceding claims, wherein the composition comprises:
i.) thymol;
ii.) carvone;
iii.) decyl glucoside;
iv.) 1, 3-propanediol;
v.) sorbitol;
vi.) glycerin;
vii.) xylitol;
viii.) lactic acid; and
vii. water.

10. The personal care composition of any of the preceding claims, wherein the essential oil system comprises a ratio (by wt.%) of thymol (by wt.%) to carvacrol (by wt.%) of 5:1 to 1:5.

11. The personal care composition of any of the preceding claims, wherein the pH of the composition is from 3 - 5.

12. The personal care composition of any of the preceding claim, wherein the composition is selected from: a hand sanitizer, a liquid soap, liquid hand soap, shower gel, body wash, shampoo, and hair conditioner.

13. The personal care composition of claim 12, wherein the composition is a hand sanitizer.

14. The personal care composition of any preceding claims, wherein the composition comprises thymol and carvone, and either citric acid or lactic acid, and wherein the composition reduces a *E. coli* population by at least 95% after about 30 seconds or 60 seconds of contact.

15. The personal care composition of any of claims 1- 14 for use in a method of reducing topical bacteria on a subject's skin, wherein the method comprises administering the personal care composition to the subject's skin and wherein the personal care composition reduces the amount of *E. coli* and/or *S. aureus* bacteria.

## Patentansprüche

1. Körperpflegezusammensetzung, die Folgendes umfasst:
a. eine wirksame Menge eines ätherischen Ölsystems, wobei das ätherische Ölsystem Thymol, Carvon und ein optionales ätherisches Öl umfasst, das unter Eugenol, Carvacrol, Teebaumöl und Kombinationen davon ausgewählt ist; und
b. eine Carbonsäurequelle, wobei die Carbonsäurequelle Milchsäure und/oder Citronensäure umfasst.

2. Körperpflegezusammensetzung nach Anspruch 1, wobei das ätherische Ölsystem Folgendes umfasst: Thymol, Carvon und Eugenol; Thymol, Carvon und Teebaumöl; Thymol, Carvon und Carvacrol; Thymol, Carvon, Eugenol und Teebaumöl; oder Thymol, Carvon, Carvacrol, Eugenol und Teebaumöl.

3. Körperpflegezusammensetzung nach einem der vorhergehenden Ansprüche, wobei das ätherische Ölsystem Teebaumöl umfasst und die Carbonsäurequelle Milchsäure umfasst.

4. Körperpflegezusammensetzung nach einem der vorhergehenden Ansprüche, wobei das Thymol in einer Menge von 0,05 Gew.-% bis 2 Gew.-%, bezogen auf die Gesamtzusammensetzung, vorliegt.

5. Körperpflegezusammensetzung nach einem der vorhergehenden Ansprüche, wobei das ätherische Ölsystem in einer Menge von 0,075 Gew.-% bis 4 Gew.-%, bezogen auf die Gesamtzusammensetzung, vorliegt, insbesondere von 0,075 Gew.-% bis 0,2 Gew.-%, genauer etwa 0,15 Gew.-%.

6. Körperpflegezusammensetzung nach einem der vorhergehenden Ansprüche, die ein Tensid umfasst, wobei das Tensid ein Alkylglucosid umfasst, und/oder wobei die Carbonsäurequelle Milchsäure umfasst.

7. Körperpflegezusammensetzung nach Anspruch 6, wobei das Alkylglucosid, falls vorhanden, Decylglucosid ist.

8. Körperpflegezusammensetzung nach Anspruch 7, wobei die Milchsäure, falls vorhanden, in einer Menge von 0,05 Gew.-% bis 2 Gew.-% der Zusammensetzung vorliegt.

9. Körperpflegezusammensetzung nach einem der vorhergehenden Ansprüche, wobei die Zusammensetzung Folgendes umfasst:
i.) Thymol;
ii.) Carvon;
iii.) Decylglucosid;
iv.) 1,3-Propandiol;
v.) Sorbitol;
vi.) Glycerin;
vii.) Xylitol;
viii.) Milchsäure; und
vii. Wasser.

10. Körperpflegezusammensetzung nach einem der vorhergehenden Ansprüche, wobei das ätherische Ölsystem ein Verhältnis (in Gew.-%) von Thymol (in Gew.-%) zu Carvacrol (in Gew.-%) von 5:1 bis 1:5 aufweist.

11. Körperpflegezusammensetzung nach einem der vorhergehenden Ansprüche, wobei der pH-Wert der Zusammensetzung 3 bis 5 beträgt.

12. Körperpflegezusammensetzung nach einem der vorhergehenden Ansprüche, wobei die Zusammensetzung ausgewählt ist unter einem Handdesinfektionsmittel, einer Flüssigseife, einer flüssigen Handseife, einem Duschgel, einem Körperwaschmittel, einem Shampoo und einer Haarspülung.

13. Körperpflegezusammensetzung nach Anspruch 12, wobei die Zusammensetzung ein Handdesinfektionsmittel ist.

14. Körperpflegezusammensetzung nach einem der vorhergehenden Ansprüche, wobei die Zusammensetzung Thymol und Carvon sowie entweder Citronensäure oder Milchsäure umfasst, und wobei die Zusammensetzung eine *E*. *coli*-Population nach einer Kontaktzeit von etwa 30 Sekunden oder 60 Sekunden um mindestens 95 % reduziert.

15. Körperpflegezusammensetzung nach einem der Ansprüche 1 bis 14 zur Verwendung in einem Verfahren zum Reduzieren topischer Bakterien auf der Haut eines Subjekts, wobei das Verfahren das Verabreichen der Körperpflegezusammensetzung auf die Haut des Subjekts aufweist und wobei die Körperpflegezusammensetzung die Menge an *E*. *coli-* und/oder *S. aureus-*Bakterien reduziert.

## Revendications

1. Composition de soins personnels comprenant :
a. une quantité efficace d'un système d'huiles essentielles, dans lequel le système d'huiles essentielles comprend du thymol, de la carvone et une huile essentielle facultative choisie dans le groupe constitué par l'eugénol, le carvacrol, l'huile essentielle d'arbre à thé et des combinaisons de ceux-ci ; et
b. une source d'acide carboxylique, et dans laquelle la source d'acide carboxylique est l'acide lactique et/ou l'acide citrique.

2. Composition de soins personnels selon la revendication 1, dans laquelle le système d'huiles essentielles comprend : du thymol, de la carvone et de l'eugénol ; du thymol, de la carvone et de l'huile essentielle d'arbre à thé ; du thymol, de la carvone et du carvacrol ; du thymol, de la carvone, de l'eugénol et de l'huile essentielle d'arbre à thé ; ou du thymol, de la carvone, du carvacrol, de l'eugénol et de l'huile essentielle d'arbre à thé.

3. Composition de soins personnels selon l'une quelconque des revendications précédentes, dans laquelle le système d'huile essentielle comprend de l'huile essentielle d'arbre à thé et la source d'acide carboxylique comprend de l'acide lactique.

4. Composition de soins personnels selon l'une quelconque des revendications précédentes, dans laquelle le thymol est présent en une quantité comprise entre 0,05 % et 2 % en poids de la composition totale.

5. Composition de soins personnels selon l'une quelconque des revendications précédentes, dans laquelle le système d'huiles essentielles est présent en une quantité comprise entre 0,075 et 4 % en poids de la composition totale, en particulier entre 0,075 et 0,2 %, plus particulièrement autour de 0,15 %.

6. Composition de soins personnels selon l'une quelconque des revendications précédentes, comprenant un tensioactif, dans laquelle le tensioactif comprend un alkylglucoside, et/ou dans laquelle la source d'acide carboxylique comprend de l'acide lactique.

7. Composition de soins personnels selon la revendication 6, dans laquelle l'alkylglucoside, s'il est présent, est un décylglucoside.

8. Composition de soins personnels selon la revendication 7, dans laquelle l'acide lactique, s'il est présent, est présent à raison de 0,05 % à 2 % en poids de la composition.

9. Composition de soins personnels selon l'une quelconque des revendications précédentes, dans laquelle la composition comprend :
i.) du thymol ;
ii.) de la carvone ;
iii.) un décyl glucoside ;
iv.) du 1,3-propanediol ;
v.) du sorbitol ;
vi.) de la glycérine ;
vii.) du xylitol ;
viii.) de l'acide lactique ; et
vii) de l'eau.

10. Composition de soins personnels selon l'une quelconque des revendications précédentes, dans laquelle le système d'huiles essentielles présente un rapport (en % en poids) du thymol (en % en poids) au carvacrol (en % en poids) de 5:1 à 1:5.

11. Composition de soins personnels selon l'une quelconque des revendications précédentes, dans laquelle le pH de la composition est compris entre 3 et 5.

12. Composition de soins personnels selon l'une quelconque des revendications précédentes, dans laquelle la composition est choisie parmi : un désinfectant pour les mains, un savon liquide, un savon liquide pour les mains, un gel douche, un nettoyant pour le corps, un shampooing et un après-shampooing.

13. Composition de soins personnels selon la revendication 12, dans laquelle la composition est un désinfectant pour les mains.

14. Composition de soins personnels selon l'une quelconque des revendications précédentes, dans laquelle la composition comprend du thymol et de la carvone, et soit de l'acide citrique soit de l'acide lactique, et dans laquelle la composition réduit une population de *E*. *coli* d'au moins 95 % après environ 30 secondes ou 60 secondes de contact.

15. Composition de soins personnels selon l'une quelconque des revendications 1 à 14, pour une utilisation dans un procédé de réduction des bactéries topiques sur la peau d'un sujet, dans laquelle le procédé comprend l'administration de la composition de soins personnels à la peau du sujet et dans lequel la composition de soins personnels réduit la quantité de bactéries *E*. *coli* et/ou *S. aureus.*
